# EUROPEAN PATENT APPLICATION

(11) **EP 2 364 703 A1**
(43) Date of publication of application: **14.09.2011**
(21) Application number: 10305252.8
(22) Date of filing: 12.03.2010
(51) Int. Cl.: A61K 31/343, A61P 9/06

(54) **Use of dronedarone or a pharmaceutically acceptable salt thereof, for the preparation of a medicament for administration shortly after amiodarone discontinuation**

(71) Applicant: SANOFI, 75013 Paris (FR)
(72) Inventor: The designation of the inventor has not yet been filed
(74) Representative: Bouron, Estelle

(57) **Abstract**

The present invention concerns the use of dronedarone or a pharmaceutically acceptable salt thereof, for the preparation of a medicament for use in the prevention and/or treatment of patients with atrial fibrillation (AF) or atrial Flutter (AFL), shortly after discontinuation of a previous treatment with amiodarone.

## Description

The present invention relates to the use of dronedarone or a pharmaceutically acceptable salt thereof, for the preparation of a medicament for use in the prevention or treatment of atrial fibrillation (usually abbreviated to AF) and/or atrial Flutter (usually abbreviated to AFL) shortly after discontinuation of a previous treatment with amiodarone.

Restoration and maintenance of sinus rhythm remain major therapeutic goals for patients with atrial fibrillation.

Amiodarone is an especially potent atrial antifibrillatory agent, which is effective in maintaining sinus rhythm in atrial fibrillation but it is also associated with potentially serious toxic effects in some patients.

Dronedarone is a new antiarrhythmic agent on the market, pharmacologically related to amiodarone but developed to reduce the risk of side effects. Indeed, dronedarone is a benzofuran derivative with an electropharmacologic profile closely resembling that of amiodarone but with structural differences intended to eliminate the effects of amiodarone on thyroid and pulmonary functions. 2-n-Butyl-3-[4-(3-di-n-butylaminopropoxy)benzoyl]-5-methylsulphonamidobenzofuran, or dronedarone, and pharmaceutically acceptable salts thereof, in particular its hydrochloride salts, are described in European Patent EP 0 471 609 B1. The elimination half-life of dronedarone is about 1 to 2 days.
Moreover, dronedarone is effective in maintaining sinus rhythm in patients presenting atrial fibrillation or atrial flutter.

The use of drugs that are devoid of such effects but that retain the antiarrhythmic potential of amiodarone are therapeutically sought.
Thus, Dronedarone, a new multichannel blocker for atrial fibrillation/flutter (AF/AFL), may be indicated for patients on prior amiodarone.

However, physicians may hesitate to start other antiarrhythmic drugs after amiodarone intake, given that the elimination half-life of amiodarone is long, between 28 to 180 days, in general between 30 to 55 days, and that tissue accumulation can cause severe organ toxicity.

So, it is of the utmost importance to find how to switch therapeutic treatment after the use of amiodarone by a patient in order to use dronedarone without harmful effects for said patient, given the long elimination half-life of amiodarone and the risk of potential additive effects (ex: QT prolongation, bradicardia).

To solve this problem, the applicant has evaluated the effect of dronedarone start after amiodarone discontinuation.

Now, the applicant surprisingly found that dronedarone or a pharmaceutically acceptable salt thereof, can be used for the preparation of a medicament for use in the prevention and/or treatment of patients with cardiovascular history, shortly after the discontinuation of a previous treatment with amiodarone.

The meaning of "shortly after the discontinuation of a previous treatment with amiodarone" may be defined as a period lower than five elimination half-lives of amiodarone, for example one elimination half-life, and more particularly four weeks, two weeks or 2 days after amiodarone discontinuation or even 1 day after said discontinuation.

Said dronedarone or pharmaceutically acceptable salt thereof, can be taken by these patients within a period lower than five elimination half-lives of amiodarone, for example, within four weeks, two weeks or 2 days after amiodarone discontinuation or even within 1 day after said discontinuation.

The patients concerned by the present invention have a cardiovascular history. They may be affected, for example, by a Atrial Fibrillation such as a persistent Atrial Fibrillation, a paroxysmal Atrial Fibrillation or a permanent Atrial Fibrillation or by a Atrial Flutter.

According to one embodiment, patients are chosen from patients with a persistent Atrial Fibrillation and in particular patients for whom cardioversion, anti-arrhythmic treatment and anticoagulation treatment are indicated.

According to another embodiment, patients are chosen from patients with paroxysmal or persistent atrial fibrillation.

According to the invention, patients may have received a previous treatment with amiodarone for at least 28 days + or - 2 days or for at least 2 months or for at least 6 months before said discontinuation.

According to one embodiment, this previous treatment with amiodarone may be short. A short-term treatment / regimen may be defined as a treatment lower than six months, for example may be for at least 28 days + or - 2 days or for at least 2 months before said discontinuation.

According to another embodiment, this previous treatment with amiodarone may be long. A long-term treatment / regimen of amiodarone may be defined as a treatment of at least 6 months before said discontinuation.

The American College of Cardiology, American Heart Association, and the European Society of Cardiology recommend in their guidelines the following classification system based on simplicity and clinical relevance:
➢ Patients with Paroxysmal Atrial Fibrillation means patients with recurrent episodes that self-terminate in less than 7 days.
➢ Patients with persistent Atrial Fibrillation means patients with recurrent episodes that last more than 7 days.

If a first detected episode self-terminates in less than 7 days and then another episode begins later on, the case has moved into the category of paroxysmal AF. Although patients in this category have episodes lasting up to 7 days, in most cases of paroxysmal AF the episodes will self-terminate in less than 24 hours. If instead the episode lasts for more than 7 days, it is unlikely to self-terminate and it is called persistent AF. In this case, the episode may be terminated by cardioversion.
➢ If cardioversion is unsuccessful or it is not attempted, and the episode is ongoing for a long time (e.g. a year or more), the patient's AF is called permanent.

Among the patients having a cardiovascular history according to the invention, in particular having a history of atrial fibrillation or atrial flutter, mention may also be made of patients also exhibiting at least one additional risk factors, such as:
- age equal to or above 70, or even above 75
- hypertension,
- diabetes,
- prior cerebrovascular disease,
- left atrial diameter greater than or equal to 50 mm measured by echocardiography,
- left ventricular ejection fraction less than 40%, measured by two-dimensional echography.

Among the patients having a cardiovascular history according to the invention, in particular having a history of atrial fibrillation or atrial flutter, mention may also be made of patients also exhibiting additional risk factors, i.e. at least one pathologies chosen from :
- Structural heart disease,
- Lone Atrial Fibrillation,
- Coronary heart disease, and
- Dilated cardiomyopathy,
and/or at least one cardiac device chosen from:
- Pacemaker, and
- Valvular heart.

Among the patients having a cardiovascular history according to the invention, in particular having a history of atrial fibrillation or atrial flutter, mention may also be made of patients also taking in at least a baseline medication chosen from:
- oral anticoagulant (usually abbreviated to OAC),
- Beta blocking agents,
- Angiotensin I converting enzyme (usually abbreviated to ACE) or Angiotensin II receptor blocker (usually abbreviated to ARB),
- Chronic antiplatelet therapy,
- Diuretics,
- Statins,
- Digitalis,
- Moderate inhibitors of CYP3A4,
- Nonsteroidal anti-inflammatory drugs (usually abbreviated to NSAID).

For their therapeutic use, dronedarone and pharmaceutically acceptable salts thereof are generally introduced into pharmaceutical compositions.

These pharmaceutical compositions contain an effective dose of dronedarone or of a pharmaceutically acceptable salt thereof, and also at least one pharmaceutically acceptable excipient.

Said excipients are chosen according to the pharmaceutical form and the method of administration desired, from the usual excipients which are known to those skilled in the art.

In said pharmaceutical compositions for oral, sublingual, subcutaneous, intramuscular, intravenous, topical, local, intratracheal, intranasal, transdermal or rectal administration, dronedarone, or the salt thereof, can be administered in unit administration form, as a mixture with conventional pharmaceutical excipients, to animals and to humans in the cases mentioned above.

The suitable unit administration forms comprise forms for oral administration, such as tablets, soft or hard gel capsules, powders, granules and oral solutions or suspensions, sublingual, buccal, intratracheal, intraocular or intranasal administration forms, forms for administration by inhalation, topical, transdermal, subcutaneous, intramuscular or intravenous administration forms, rectal administration forms, and implants. For topical application, dronedarone and pharmaceutically acceptable salts thereof can be used in creams, gels, ointments or lotions.

By way of example, a unit administration form of dronedarone or a pharmaceutically acceptable salt thereof, in tablet form, may correspond to one of the following compositions (Examples 1 - 4) according to the invention:

| **EXAMPLE 1** | |
|---|---|
| Ingredients | mg |
| Dronedarone hydrochloride (corresponding to 400 mg of base) | 426 |
| Methylhydroxypropylcellulose | 21.1 |
| Lactose monohydrate | 46.55 |
| Maize starch | 45.5 |
| Polyvinylpyrrolidone | 65 |
| Poloxamer 407 | 40 |
| Anhydrous colloidal silica | 2.6 |
| Magnesium stearate | 3.25 |
| | 650 |
| | |

| **EXAMPLE 2** | |
|---|---|
| Ingredients | mg |
| Dronedarone hydrochloride (corresponding to 400 mg of base) | 426 |
| Microcrystalline cellulose | 65 |
| Anhydrous colloidal silica | 2.6 |
| Anhydrous lactose | 42.65 |
| Polyvinylpyrrolidone | 13 |
| Poloxamer 407 | 40 |
| Macrogol 6000 | 57.5 |
| Magnesium stearate | 3.25 |
| | 650 |

| **EXAMPLE 3** | |
|---|---|
| Ingredients | mg |
| Dronedarone hydrochloride (corresponding to 400 mg of base) | 426 |
| Microcrystalline cellulose | 26 |
| Maize starch | 45.5 |
| Polyvinylpyrrolidone | 65 |
| Poloxamer 407 | 40 |
| Anhydrous colloidal silica | 3.25 |
| Magnesium stearate | 3.25 |
| Lactose monohydrate | 41.65 |
| | 650 |
| | |

| **EXAMPLE 4** | |
|---|---|
| Ingredients | mg |
| Dronedarone hydrochloride (corresponding to 400 mg of base) | 213 |
| Microcrystalline cellulose | 13 |
| Maize starch | 22.75 |
| Polyvinylpyrrolidone | 32.5 |
| Poloxamer 407 | 20 |
| Anhydrous colloidal silica | 1.3 |
| Magnesium stearate | 1.625 |
| Lactose monohydrate | 20.825 |
| | 650 |

The dose of dronedarone administered per day, orally, may reach 800 mg, taken in one or more intakes. More specifically, the dose of dronedarone administered may be taken with food. For example, the dose of dronedarone administered per day, orally, may reach 800 mg, taken in two intakes with a meal.

The dose of dronedarone administered per day, orally may be taken at a rate of twice a day (usually abbreviated BID) with a meal for example with the morning and the evening meal. More specifically, the two intakes may comprise same quantity of dronedarone.

Advantageously, the dose of dronedarone administered, orally, may reach 400 mg BID, taken together with a meal, for example with the morning and the evening meal.

There may be specific cases where higher or lower dosages are appropriate; such dosages do not depart from the context of the invention. According to the usual practice, the dosage appropriate for each patient is determined by the physician according to the method of administration, the weight, the pathology, the body surface, the cardiac output and the response of said patient.

According to another of its aspects, the present invention also relates to a method for treating the pathologies indicated above, which comprises the administration, to a patient, of an effective dose of dronedarone or a pharmaceutically acceptable salt thereof.

Amiodarone treatment may be stopped directly or the administered dose of amiodarone may decrease progressively before its discontinuation. More particularly, this dose of amiodarone may decrease from a loading dose to a maintenance dose for example from about 600 mg to about 200 mg per day, orally, taken in one or more intakes.

According to one embodiment, the administered dose of amiodarone is 600 mg daily for about 1 week, then 400 mg daily for about 1 week, and finally 200 mg daily for about 2 weeks before discontinuation.

According to another embodiment, the administered dose of amiodarone is a maintenance dose for example about 200 mg daily. This maintenance dose may be administrated for a defined period before discontinuation for example at least two months before discontinuation.

The objectives of the present study is to show the effects of dronedarone start on patients having a cardiovascular history, indeed said patients being previously treated with amiodarone and, in particular, the effects of treatment change after a variable duration of wash-out between the two drugs periods.

**Methods:** In EURIDIS and ADONIS studies mentioned in the article of the New England Journal of Medicine , 357, 10, September 6, 12007), 828 and 409 AF/AFL patients were randomized after conversion to SR to dronedarone (400 mg BID) or placebo for 1 year.

During this study, a subgroup (average age: 63.5 years, 35.7% of women) of 98 patients treated with dronedarone and 56 patients treated with the placebo were switched from amiodarone to dronedarone or placebo within 48 hours.

**Results:** Dronedarone decreased AF/AFL recurrence compared to placebo (HR=0.64 [95% Cl 0.44-0.95], P=0.022) in this subgroup. Rates of adverse events (abbreviated AEs), specifically bradyarrhythmia, in this subgroup were compared to 334 patients treated with placebo and 680 patients treated with dronedarone (average age: 62.8 years; 29.7% of women) with no previous amiodarone use and a smaller group of 17 and 35 patients (average age: 65.5 years; 30.8% of women) switched after 48 h of discontinuation. All the results are included in the table 1 below.

**TABLE 1**

| **Events** | **A stopped within 2 days before randomization** | | **A stopped more than 2 days before randomization** | | **No amiodarone treatment before randomization** | |
|---|---|---|---|---|---|---|
| **Drug** | **PI** | **D** | **PI** | **D** | **PI** | **D** |
| **Tested population** | **n=56** | **n=98** | **n=17** | **n=35** | **n= 334** | **n=680** |
| **Deaths (n)** | **1** | **1** | **1** | **2** | **2** | **5** |
| **Rate of Serious adverse events (%)** | **14.3** | **15.3** | **11.8** | **17.1** | **16.2** | **13.7** |
| | | | | | | |
| **Bradyarrhythmia(%)** | **0** | **1** | **0** | **0** | **0.9** | **1.3** |
| **Rate of Adverse Events with drug discontinuation (%)** | **3.6** | **8.2** | **11.8** | **20.0** | **6.3** | **9.1** |
| | | | | | | |
| **Bradyarrhythmia(%)** | **0** | **3.1** | **0** | **0** | **0** | **0.7** |
| **PR-interval >or = 200 msec and increase >or = 20 msec versus baseline** | **14.5** | **25.8** | **12.5** | **22.9** | **12.8** | **28.0** |
| **QTc-Fridericia >or = 500 msec** | **3.6** | **7.9** | **6.7** | **14.3** | **0.3** | **4.4** |

In the table 1 above, "A" means Amiodarone, "D" means" dronedarone, "Pl" means Placebo, "n" is the number of patients tested with "Pl" or "D".

There were slightly more bradyarrhythmic events leading to drug discontinuation and numerically more QTc-prolongations in switch patients, as expected from the pharmacodynamic profile of the drugs. Rate of serious AEs was low and similar across groups with no episodes of torsades de pointes.

**Conclusions:** This post hoc analysis of a small patient cohort switched rapidly from A to D resulted in good tolerability.

Further to these results, another study has been initiated to evaluate the optimal timing of dronedarone treatment start after conversion with loading dose of amiodarone in patients with a cardiovascular history, in particular patients with persistent atrial fibrillation requiring Atrial Fibrillation conversion.

For that, the study primary objectives are to evaluate the rate of AF recurrences one month after randomization according to different timings of initiation of dronedarone hydrochloride.

The study design concerns international, prospective, multicenter, open-label study, randomized in 3 parallel groups according to dronedarone treatment initiation patterns following a loading dose of amiodarone in patients with persistent atrial fibrillation requiring AF conversion.

### I. PATIENT SELECTION

### I.1. Main inclusion criteria of the patients at screening:

- Male or female,
- Patients with persistent AF for more than 48 hours (documented by an ECG taken within the last 48 hours) for whom cardioversion, anti-arrhythmic treatment and anticoagulation treatment are indicated,
- Naive of amiodarone treatment in the last three months prior to screening,
- QTcB < 500 ms on 12-lead ECG,
- Patient with at least one cardiovascular risk factor (i.e. age > 70, hypertension, diabetes, prior cerebrovascular disease, left atrial diameter≥ 50 mm or left ventricular ejection fraction [LVEF] < 40%).

### I.2. Main inclusion criteria of the patients before randomization:

- Outpatients (only hospitalization of 48h for a planned cardio-version is allowed),
- Patients in sinus rhythm,
- Patients under effective oral anticoagulation according to ACC/AHA/ESC 2006 guidelines,
- QTcB < 500 ms and PR < 280 ms on 12-lead ECG,
- Patients having received 28 days ± 2 days of amiodarone.

### I.3. Main general exclusion criteria at screening (S) and randomization (R):

- Contraindication to oral anticoagulation (S),
- Any documented AF episode motivating inclusion in the study after an acute condition known to cause AF (S),
- Permanent AF in which cardioversion has failed (S),
- Paroxysmal AF (S),
- Bradycardia < 50 bpm on the 12-lead ECG (S, R),
- Clinically overt congestive heart failure (S, R):
   o with New York Heart Association (NYHA) classes III and IV heart failure,
   o or NYHA class II with a recent decompensation requiring hospitalization or referral to a specialized heart failure clinic,
   o as well as any patients in unstable hemodynamic conditions,
- Previous treatment with class I or class III anti-arrhythmic drugs (including sotalol) if taken less than one week before screening (S, R),
- Previous history of amiodarone intolerance or toxicity (S),
- Any contraindication as per dronedarone and amiodarone labelling (S, R),
- Wolff-Parkinson-White Syndrome (S),
- Previous ablation for atrial fibrillation or any planned ablation in the next 2 months (S).

### I.4. Main exclusion criteria related to concomitant medications:

Patients in whom a contraindicated concomitant treatment is mandatory (refer to protocol body for more details), said treatment including:
   o use of potent cytochrome P450 (CYP3A4) inhibitors,
   o concomitant use of drugs or herbal products that prolong the QT interval and known to increase the risk of Torsades de Pointes,
   o use of class I or III anti-arrhythmic drugs (including sotalol).

### II. DURATION, SCHEDULE AND TREATMENT

### II. 1. Duration of study participation:

Patients are screened for a maximum of 10 weeks and a minimum of 5 weeks prior to randomization. After randomization, patients are treated with dronedarone for 8, 6 or 4 weeks (according to the duration of the amiodarone discontinuation) until the end of treatment (EOT)/end of study visit (EOS), visit occurring at Week 8 after randomization.

### II.2. Assessment schedule:

Patients have regular visits (V) during:

### PHASE I: Screening phase : maximum of 3 visits

- **Screening (V1)** (up to maximum of Week -10 (Day -70) if no previous anticoagulation to minimum of Week -5 (Day -35) if patient already under effective anticoagulation at screening),
- **Initiation of amiodarone (V2)** (Day -28 ± 2 days),
- **Electrical cardioversion (V3)** (allowed after 7 days of amiodarone treatment and up to Day 1 included). This visit is optional and the electrical cardioversion is only required if the patient is still in AF.

### PHASE II: Randomization (V4) (Day 1, maximum of 10 weeks and minimum of 5 weeks after screening). After randomization, start of dronedarone in group A and start of wash-out periods in groups B and C.

During the randomized phase, there is a maximum of 6 visits per arm after randomization:
o **V5**: start of dronedarone
o **V6**: after 2 days of dronedarone
o **V7:** after 1 week of dronedarone
o **V8**: after 2 weeks of dronedarone
o **V9**: after 4 weeks of dronedarone

**V10 = End of treatment visit/end of study visit:** after 8, 6 or 4 weeks of dronedarone according to the duration of amiodarone discontinuation.

### PHASE III: Results

Primary Endpoint are assessed at 4 weeks after randomization.

### II.3. Treatment:

Patients are randomized in three parallel groups in which the timing of initiation of dronedarone after the loading regimen of amiodarone differs, namely:
➢ immediate start of dronedarone in the **group A**, and
➢ start after more than 2 days, (i.e. after a 2-week or 4-week wash-out) in the **groups B** and **C** respectively.

Treatment is initiated using tablets containing either 400 mg of dronedarone or tablets containing 200 mg of amiodarone.

The dose regimen is:
(i) for dronedarone: 400 mg BID, and
(ii) for amiodarone: 600 mg daily for 1 week, 400 mg daily for 1 week and 200 mg daily for 2 weeks.

Another study has been initiated to evaluate the optimal timing of dronedarone treatment start after discontinuation of amiodarone in patients with a cardiovascular history, in particular patients with paroxysmal or persistent atrial fibrillation and wherein said patients have received at least 6 months of amiodarone.

For that, the study primary objectives are to explore dronedarone and its active metabolite PK profiles according to different timing of dronedarone initiation and secondary objectives are to explore potential PK interaction between dronedarone and amiodarone, to evaluate the rate of AF recurrence one month and two months after randomization and to assess the safety of the change from amiodarone to dronedarone and dronedarone safety.

The study design concerns international, prospective, multicenter, open-label study, randomized in 3 parallel groups according to dronedarone treatment initiation patterns following long-term regimen of amiodarone in patients with paroxysmal or persistent atrial fibrillation.

### I. PATIENT SELECTION

### I.1. Main inclusion criteria of the patients at screening:

- Male or female,
- Outpatients,
- Patients with paroxysmal or persistent AF having received at least 6 months of amiodarone before screening with at least the last 2 months at a regimen of 200 mg/day prior screening,
- Requiring a change from amiodarone treatment whatever the reason, but without major amiodarone-related toxicity (interstitial lung disease, thyroid or hepatotoxicity).
- QTcB < 500 ms on 12-lead ECG,
- Patient with at least one cardiovascular risk factor (i.e. age > 70, hypertension, diabetes, prior cerebrovascular disease, left atrial diameter ≥ 50 mm or left ventricular ejection fraction [LVEF] < 40%),

### I.2. Main inclusion criteria of the patients before randomization:

- Outpatients (only hospitalization of 48h for a planned cardio-version will be allowed)
- Patients in sinus rhythm before randomization,
- Patients under effective oral anticoagulation according to ACC/AHA/ESC 2006 guidelines,
- QTcB < 500 ms and PR < 280 ms on 12-lead ECG.

### 1.3. Main general exclusion criteria at screening (S) and randomization (R):

- Contraindication to oral anticoagulation,
- Any documented AF episode motivating inclusion in the study after an acute condition known to cause AF,
- Permanent AF in which cardioversion has failed,
- Bradycardia < 50 bpm on the 12-lead ECG before randomization,
- Clinically overt congestive heart failure:
   o with New York Heart Association (NYHA) classes III and IV heart failure,
   o or NYHA class II with a recent decompensation requiring hospitalization or referral to a specialized heart failure clinic,
   o as well as any patients in unstable hemodynamic conditions
- Previous treatment with class I or class III anti-arrhythmic drugs (including sotalol) if taken less than one week before,
- Previous history of amiodarone intolerance or toxicity,
- History of thyroid dysfunction,
- Any contraindication as per dronedarone labelling,
- Wolff-Parkinson-White Syndrome,
- Previous catheter ablation for atrial fibrillation,
- Catheter ablation scheduled in the next 10 weeks
- Patients previously treated with class I or class III anti-arrhythmic drugs other than amiodarone if the anti-arrhythmic drug was taken less than one week before the day of screening (If taken more than one week before screening, the patient can be included).

### I.4. Main exclusion criteria related to concomitant medications:

Patients in whom a contraindicated concomitant treatment is mandatory (refer to protocol body for more details), said treatment including:
o use of potent cytochrome P450 (CYP3A4) inhibitors,
o concomitant use of drugs or herbal products that prolong the QT interval and known to increase the risk of Torsades de Pointes,
o use of class I or III anti-arrhythmic drugs (including sotalol).

### II. DURATION, SCHEDULE AND TREATMENT

### II. 1. Duration of study participation:

Patients can be screened for a maximum of 10 days and a minimum of 3 days prior to randomization. After randomization, patients will be treated with dronedarone for 8, 6 or 4 weeks (according to the group) until the end of treatment (EOT)/end of study visit (EOS) visit occurring at Week 8 after randomization.

After the last dose of dronedarone, the investigator will be free to continue with the treatment of his choice.

### II.2. Assessment schedule:

Patients have regular visits (V) during:

### PHASE I: Screening phase :

- **Screening visit (V1)**: Day-10 to Day-3 before randomization. Patient will remain on amiodarone at a regimen of 200mg/day until randomization.
- **Electrical cardioversion (V2,** allowed from screening to Day 1 included). This visit is optional, and the electrical cardioversion is only required if the patient is still in AF

### PHASE II: Randomization (V3) (Day1. After randomization, start of dronedarone in group A and start of washout periods in groups B and C).

During the randomized phase, there is a maximum of 6 visits per arm after randomization:
o **V4**: start of dronedarone
o **V5**: after 2 days of dronedarone
o **V6**: after 1 week of dronedarone
o **V7**: after 2 weeks of dronedarone
o **V8**: after 4 weeks of dronedarone
o **V9 = End of treatment visit (EOT)/ end of study visit (EOS):** after 8, 6 or 4 weeks of dronedarone according to treatment group

### II.3. Treatment:

Patients are randomized in three parallel groups in which the timing of initiation of dronedarone after discontinuation of amiodarone treatment differs, namely:
➢ immediate start of dronedarone in the **group A**, and
➢ start after more than 2 days, (i.e. after a 2-week or 4-week wash-out) in the **groups B** and **C** respectively.

Treatment is initiated using tablets containing either 400 mg of dronedarone or tablets containing 200 mg of amiodarone.

The dose regimen is:
(iii) for dronedarone: 400 mg BID, and
(iv) for amiodarone: 200 mg daily.

## Claims

1. Use of dronedarone or a pharmaceutically acceptable salt thereof, for the preparation of a medicament for use in the prevention and/or treatment of patients with atrial fibrillation (AF) or atrial Flutter (AFL), shortly after discontinuation of a previous treatment with amiodarone.

2. Use according to claim 1, for the preparation of a medicament for use in the prevention and/or treatment of patients with atrial fibrillation (AF) and/or atrial Flutter (AFL) within a period lower than five elimination half-lives after said discontinuation.

3. Use according to anyone of the claims 1 or 2, for the preparation of a medicament for use in the prevention and/or treatment of patients with atrial fibrillation (AF) and/or atrial Flutter (AFL) shortly, within four weeks after said discontinuation.

4. Use according to anyone of the claims 1 to 3, for the preparation of a medicament for use in the prevention and/or treatment of patients with atrial fibrillation (AF) and/or atrial Flutter (AFL) shortly, within two weeks after said discontinuation.

5. Use according to anyone of the claims 1 to 4, for the preparation of a medicament for use in the prevention and/or treatment of patients with atrial fibrillation (AF) or atrial Flutter (AFL) within two days after said discontinuation.

6. Use according to anyone of the claims 1 to 5, for the preparation of a medicament for use in the prevention and/or treatment of patients with atrial fibrillation (AF) and/or atrial Flutter (AFL) within one day after said discontinuation.

7. Use according to anyone of the preceding claims, for the preparation of a medicament for use in the prevention and/or treatment of patients with persistent Atrial Fibrillation.

8. Use according to the preceding claim, **characterized in that** patients with a persistent Atrial Fibrillation are chosen from patients for whom cardioversion, anti-arrhythmic treatment and anticoagulation treatment are indicated.

9. Use according to anyone of the claims 1 to 6, for the preparation of a medicament for use in the prevention and/or treatment of patients with paroxysmal Atrial Fibrillation.

10. Use according to anyone of the claims 1 to 6, for the preparation of a medicament for use in the prevention and/or treatment of patients with permanent Atrial Fibrillation.

11. Use according to anyone of the preceding claims, **characterized in that** patients have received a previous treatment with amiodarone for at least 28 days + or- 2 days before said discontinuation.

12. Use according to anyone of the preceding claims, **characterized in that** patients have received a previous treatment with amiodarone for at least 2 months before said discontinuation.

13. Use according to anyone of the preceding claims, **characterized in that** patients have received a previous treatment with amiodarone for at least 6 months before said discontinuation.

14. Use according to anyone of the preceding claims, **characterized in that** the dose of dronedarone administered orally, is 400 mg BID.

15. Use according to anyone of the preceding claims, **characterized in that** the administered dose of amiodarone decreases progressively before its discontinuation.

16. Use according to the preceding claim, **characterized in that** the administered dose of amiodarone decreases from a loading dose to a maintenance dose.

17. Use according to anyone of the preceding claims 15 or 16, **characterized in that** the dose of amiodarone administered is about 600 mg to about 200 mg per day, orally, taken in one or more intakes.

18. Use according to anyone of the preceding claims, **characterized in that** the dose of amiodarone administered is 200 mg daily for at least two months before discontinuation.

19. Use according to anyone of the preceding claims, **characterized in that** patients also exhibit at least one additional cardiovascular risk factor chosen from:
- age equal to or above 70, or even above 75,
- hypertension,
- diabetes,
- prior cerebrovascular disease,
- left atrial diameter greater than or equal to 50 mm measured by echocardiography, and
- left ventricular ejection fraction less than 40%, measured by two-dimensional echography.

20. Use according to anyone of the preceding claims, **characterized in that** patients also exhibit at least one pathologies chosen from :
- Structural heart disease,
- Lone Atrial Fibrillation,
- Coronary heart disease, and
- Dilated cardiomyopathy,
and/or at least one cardiac device chosen from:
- Pacemaker, and
- Valvular heart.

21. Use according to anyone of the preceding claims, **characterized in that** patients also take in at least a baseline medication chosen from:
- oral anticoagulant,
- Beta blocking agents,
- Angiotensin I converting enzyme or Angiotensin II receptor blocker,
- Chronic antiplatelet therapy,
- Diuretics,
- Statins,
- Digitalis,
- Moderate inhibitors of CYP3A4,
- Nonsteroidal anti-inflammatory drugs.
